(19) Europäisches Patentamt
European Patent Office
Office européen des brevets

(11) Veröffentlichungsnummer: **0 122 442 B1**

(12) **EUROPÄISCHE PATENTSCHRIFT**

(45) Veröffentlichungstag der Patentschrift: **19.11.87**

(21) Anmeldenummer: **84102555.4**

(22) Anmeldetag: **09.03.84**

(51) Int. Cl.⁴: **C 07 D 491/06**, C 07 D 491/16, C 09 B 5/62 // (C07D491/06, 311:00, 221:00),(C07D491/16, 311:00, 235:00, 221:00)

# (54) Perylen-3,4,9,10-tetracarbonsäure-monoanhydrid-monoimid- und -monoimidazolid-Verbindungen, Verfahren zu ihrer Herstellung und ihre Verwendung.

(30) Priorität: **14.03.83 DE 3309060**

(43) Veröffentlichungstag der Anmeldung: **24.10.84 Patentblatt 84/43**

(45) Bekanntmachung des Hinweises auf die Patenterteilung: **19.11.87 Patentblatt 87/47**

(84) Benannte Vertragsstaaten: **CH DE FR GB LI**

(56) Entgegenhaltungen:
**EP - A - 0 039 482**
**FR - A - 2 456 102**

**Chemical Abstracts Band 97, Nr. 11, 13. September 1982, Columbus, Ohio, USA G.N. VOROZHTSOV et al. "Derivatives of 1,1'-binaphthyl. IX. Formation of derivatives of 3,4,9,10-perylenetetracarboxylic acid in the electrochemical reduction of 8,8'-disubstituted derivatives of 1,1'-binaphthyl-4,4', 5,5'-tatracarboxylic acid", Seite 698, Spalte 1, Abstract Nr. 91441m & Zh. Org. Khim., Band 18, Nr. 5, 1982, Seiten 1024-1033**
**Chemical Abstracts Band 93, Nr. 26, 29. Dezember 1980, Columbus, Ohio, USA Y. NAGAO et al. "Synthesis and reactions of perylenecarboxylic acid derivatives. 4. Syntheses and properties of unsymmetrical 3,4:9,10-perylenebis(dicarboximide) derivatives", Seite 91, Spalte 1, Abstract Nr. 241183n**
**Chemical Abstracts Band 91, Nr. 4, 23. Juli 1979, Columbus, Ohio, USA Y. NAGAO *et al.* "Synthesis of unsymmetrical perylenebis(dicarboximide) derivatives", Seite 93, Spalte 1, Abstract Nr. 22404r & Chem. Lett., Nr. 2, 1979, Seiten 151-154**

(73) Patentinhaber: **HOECHST AKTIENGESELLSCHAFT, Postfach 80 03 20, D-6230 Frankfurt am Main 80 (DE)**

(72) Erfinder: **Spietschka, Ernst, Dr., Kirchweg 3, D-6270 Idstein/Taunus (DE)**
Erfinder: **Tröster, Helmut, Dr., Am Erdbeerstein 44, D-6240 Königstein/Taunus (DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 122 442 B1

**Beschreibung**

Die Erfindung liegt auf dem technischen Gebiet der Herstellung von Farbstoffen und Pigmenten.

Perylen-3,4,9,10-tetracarbonsäure-monoanhydrid-monoalkylimid-Verbindungen sind bereits aus der Europäischen Patentanmeldung-Veröffentlichung Nr. 0 039 482-A bekannt, jedoch lassen sich entsprechende Monoimid-monoanhydride mit schwächer basischen Aminen als Alkylaminen nach dem dort beschriebenen Verfahren nicht oder nur mit Schwierigkeiten und äusserst geringen Ausbeuten herstellen.

Mit der vorliegenden Erfindung wurden nunmehr neue Perylen-3,4,9,10-tetracarbonsäure-monoanhydrid-monoimid-Verbindungen der allgemeinen Formel (1)

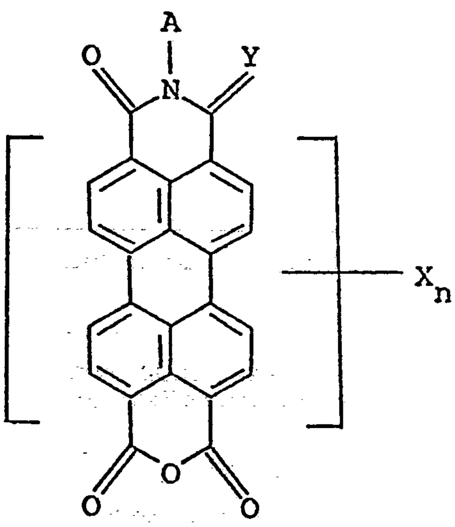
(1)

gefunden, in welcher die einzelnen Formelglieder die folgenden Bedeutungen haben: X ist ein Chlor- oder Bromatom;
n ist eine Zahl von Null bis 4;
y steht für ein Sauerstoffatom oder ein Stickstoffatom;
A ist, sofern Y für ein Sauerstoffatom steht, der Phenylrest, der durch Substituenten aus der Gruppe Halogen, wie Jod, Fluor, insbesondere Chlor und Brom, Alkyl von 1 bis 4 C-Atomen, Alkoxy von 1 bis 4 C-Atomen, Carbalkoxy mit 1 bis 4 C-Atomen im Alkylrest, wie Carbomethoxy und Carbäthoxy, Hydroxy, Carboxy, Trifluormethyl, Nitro, Phenoxy, durch Alkyl von 1 bis 4 C-Atomen, Alkoxy von 1 bis 4 C-Atomen und/oder Halogen, wie Chlor und Brom, substituiertes Phenoxy, Phenylamino, im Phenylrest durch Alkyl von 1 bis 4 C-Atomen, Alkoxy von 1 bis 4 C-Atomen und/oder Halogen, wie Chlor und Brom, substituiertes Phenylamino, Phenylazo und durch Alkyl von 1 bis 4 C-Atomen, Alkoxy von 1 bis 4 C-Atomen und/oder Halogen, wie Chlor und Brom, substituiertes Phenylazo substituiert sein kann, oder ist ein Pyridylrest, der durch Halogen, wie Brom und Chlor, und/oder Alkyl von 1 bis 4 C-Atomen, wie Äthyl und insbesondere Methyl, substituiert sein kann, oder A ist, sofern Y für ein Stickstoffatom steht, der ortho-Phenylenrest, der zusammen mit Y und dem anderen Stickstoffatom den Benzimidadol-Ring bildet, wobei dieser o-Phenylrest durch Substituenten aus der Gruppe Halogen, wie Chlor und Brom, Alkyl von 1 bis 4 C-Atomen und Alkoxy von 1 bis 4 C-Atomen substituiert sein kann.

Alkylgruppen von 1 bis 4 C-Atomen sind beispielsweise die Methyl- und die Äthylgruppe; Alkoxygruppen von 1 bis 4 C-Atomen sind beispw. die Methoxy- und die Äthoxygruppe. Die im Formelrest A befindlichen Substituenten können 1, 2 oder 3 Substituenten sein.

Bevorzugt von den Verbindungen der allgemeinen Formel (1) sind diejenigen, in welchen Y ein Sauerstoffatom und A den Phenylrest bedeutet, der durch Substituenten aus der Gruppe Alkyl von 1 bis 4 C-Atomen, Alkoxy von 1 bis 4 C-Atomen, Chlor und Brom substituiert sein kann, oder in welchen Y für ein Stickstoffatom steht und A den o-Phenylenrest bedeutet, der durch einen oder zwei Substituenten aus der Gruppe Alkyl von 1 bis 4 C-Atomen, Alkoxy von 1 bis 4 C-Atomen und Chlor substituiert sein kann.

Des weiteren sind bevorzugt diejenigen Verbindungen der allgemeinen Formel (1), in welchen n die Zahl Null bedeutet.

Die vorliegende Erfindung betrifft weiterhin ein Verfahren zur Herstellung der neuen Verbindungen der allgemeinen Formel (1), das dadurch gekennzeichnet ist, dass man das Tetrasalz der Perylen-3,4,9,10-tetracarbonsäure der allgemeinen Formel (2)

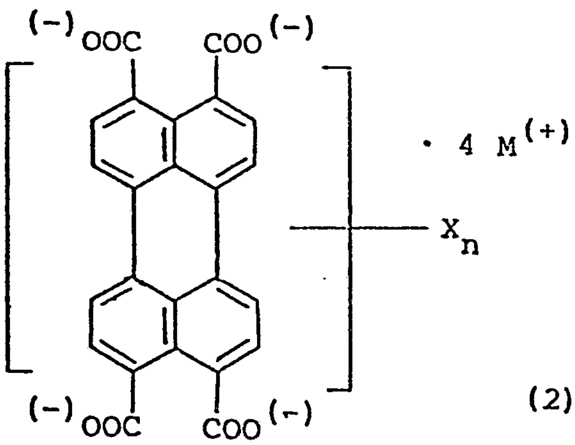
(2)

in welcher X und n die obengenannten Bedeutungen haben und $M^{(+)}$ das Ammoniumion eines sekundären oder bevorzugt tertiären Amins ist, das in wässriger Lösung einen pH-Wert von 8,5 oder höher einzustellen vermag, mit einer aromatischen Aminoverbindung der allgemeinen Formel (3) oder (4)

$B-NH_2$ (3) $H_2N-D-NH_2$ (4)

in welchen B den oben für A genannten, gegebenenfalls substituierten Phenyl- oder Pyridyl-Rest bedeutet und D den ortho-Phenylenrest darstellt, der, wie oben für A angegeben, durch Substituenten aus der Gruppe Halogen, Alkyl von 1 bis 4 C-Atomen und Alkoxy von 1 bis 4 C-Atomen substituiert sein kann, in wässriger oder wässrig-organischer Lösung miteinander umsetzt und hierbei diese Reaktionslösung bei Raumtemperatur oder erhöhter Temperatur, wie bei einer Temperatur zwischen 10° C und Siedetemperatur des Reaktionsgemisches, vorzugsweise zwischen 15 und 95° C, zunächst durch Zugabe von Säure, vorteilhaft durch allmähliche Zugabe von Säure, auf einen pH-Wert zwischen 7 und 5, vorzugsweise zwischen 6,8 und 6,2, einstellt und sodann die Umsetzung innerhalb des angebenen pH-Bereiches bei erhöhter Temperatur, vorzugsweise bei einer Temperatur zwischen 70° C und Siedetemperatur des Reaktionsmediums, wie zwischen 70 und 110° C, durchführt.

Die Umsetzung der Verbindungen der allgemeinen Formel (2) mit den Aminen der allgemeinen Formel (3) oder (4) wird unter Verwendung von äquimolaren Mengen durchgeführt, wobei ein leichter Überschuss an dem Amin (3) oder (4) zweckmässig ist. Dieser Überschuss kann, abhängig von dem als Reaktionskomponente eingesetzten Amin, insbesondere der Basizität des Amins, bis zu 20 Mol-% betragen.

Das wässrig-organische Reaktionsmedium enthält als organische Lösemittelkomponente beispielsweise Dimethylformamid oder ein ungesättigtes tertiäres Amin, wie insbesondere ein Picolin oder Pyridin. Die Ausführung des erfindungsgemässen Verfahrens in einem wässrig-organischen Reaktionsmedium mit einem Picolin oder Pyridin als organische Lösemittelkomponente stellt eine bevorzugte Ausführungsform dar. Diese ungesättigten tertiären Amine können als Puffer und Lösungsvermittler für die Reaktionskomponente im Reaktionssatz dienen; die Menge dieser organischen Lösemittelkomponente kann hierbei in weiten Grenzen variiert werden. Zweckmässig verwendet man 10 bis 80% an dem ungesättigten tertiären Amin, bezogen auf die verwendete Wassermenge der Reaktionslösung. Das ungesättigte tertiäre Amin wird vorteilhaft vor der Einstellung des pH-Bereiches zwischen 7 und 5 der wässrigen Reaktionslösung zugegeben.

Das Ammoniumion $M^{(+)}$ in den Ausgangsverbindungen der allgemeinen Formel (2) ist bevorzugt ein Ammoniumion der allgemeinen Formel (5)

$$H-\overset{(+)}{\underset{R^3}{\overset{R^1}{N}}}-R^2 \qquad (5)$$

in welcher $R^1$, $R^2$ und $R^3$ zueinander gleiche oder voneinander verschiedene Bedeutungen haben können und $R^1$ für ein Wasserstoffatom oder eine Alkylgruppe von 1 bis 4 C-Atomen, bevorzugt Methyl- oder Äthylgruppe, die durch eine Hydroxygruppe substituiert sein kann, steht, $R^2$ eine Alkylgruppe von 1 bis 4 C-Atomen, wie die Methyl- oder Äthylgruppe, bedeutet, die durch eine Hydroxygruppe substituiert sein kann, und $R^3$ eine Alkylgruppe von 1 bis 4 C-Atomen, wie die Methyl- oder Äthylgruppe, ist, die durch eine Hydroxygruppe substituiert sein kann, oder in welcher $R^2$ und $R^3$ zusammen mit dem Stickstoffatom einen gesättigten, heterocyclischen, gegebenenfalls ein Sauerstoffatom oder Stickstoffatom als weiteres Heteroatom enthaltenden Ring bilden. Entsprechende, hierfür geeignete sekundäre oder tertiäre Amine sind beispielsweise Dimethylamin, Diäthylamin, Dibutylamin, Diäthanolamin, Triäthalolamin, Trimethylamin, Triäthylamin, Piperidin und Morpholin, bevorzugt hiervon Trimethylamin und insbesondere Triäthylamin.

Aromatische Amine (3), die als Ausgangsverbindungen dienen, sind beispielsweise Anilin, 2-Methyl-anilin, 3-Methyl-anilin, 4-Methyl-anilin, 3,5-Dimethyl-anilin, 2,4-Dimethyl-anilin, 2,5-Dimethyl-anilin, 2,6-Dimethyl-anilin, 3,4-Dimethylanilin, 4-Isopropyl-anilin, 2,4,6-Trimethyl-anilin, 2-Chlor-anilin, 3-Chlor-anilin, 4-Chlor-anilin, 2,4-Dichlor-anilin, 3-Brom-anilin, 3-Jod-anilin, 2-Methyl-5-chlor-anilin, 3-Chlor-4-methyl-anilin, 3-Trifluormethyl-anilin, 3,5-Bis-(trifluormethyl)-anilin, 2-Aminophenol, 3-Aminophenol, 4-Methoxy-anilin, 4-Äthoxy-anilin, 2-Amino-1,4-dimethoxy-benzol, 4-Amino-diphenyläther, 4-Amino-4'-chlor-diphenyläther, 4-Amino-diphenylamin, 4-Amino-azobenzol, 3-Nitro-anilin, 5-Aminoisophthalsäuredimethylester, 3-Amino-benzoesäure, 2-Aminopyridin und 3-Aminopyridin.

Aminoverbindungen der allgemeinen Formel (4), die als Ausgangsverbindungen dienen, sind beispielsweise 1,2-Diaminobenzol, 4-Methyl-1,2-diaminobenzol, 4-Methoxy-1,2-diaminobenzol, 4-Chlor-1,2-diaminobenzol und 4,5-Dichlor-1,2-diaminobenzol.

Das erfindungsgemässe Verfahren kann beispielsweise in der Weise ausgeführt werden, dass man die Perylen-tetracarbonsäure oder ihr Dianhydrid zunächst in Wasser mit der zur Überführung in das Tetracarboxylat erforderlichen Menge an der Aminoverbindung, die einen pH-Wert von 8,5 oder höher in Wasser einzustellen vermag, in üblicher Weise bei erhöhter Temperatur behandelt. Zweckmässig setzt man einen geringen Überschuss, wie bis zu 10% an dem Amin ein, d.h. bis zu 4,4 Mol an diesem Amin, bezogen auf ein Mol Perylentetracarbonsäure oder -dianhydrid. Sodann gibt man das Amin der allgemeinen Formel (3) oder (4) und gegebenenfalls ein ungesättigtes tertiäres Amin, wie Pyridin, als organische Lösemittelkomponente hinzu und stellt sodann durch Zugabe von Säure einen pH-Wert innerhalb des oben erwähnten pH-Bereiches ein. Als Säuren können hierfür anorganische und organische Säuren eingesetzt werden, so beispielsweise Mineralsäuren, wie Salzsäure oder Schwefelsäure, bevorzugt jedoch wegen ihrer Pufferwirkung mittelstarke bis schwache organische oder anorganische Säuren, wie beispielsweise Ameisensäure, Essigsäure, Propionsäure, Kohlensäure oder Phosphorsäure. Der Ansatz wird sodann erwärmt, und der angegebene pH-Bereich durch Zugabe von weiterer Säure gehalten, da der pH-Wert während der Reaktion andernfalls ansteigt.

Das gebildete Reaktionsprodukt wird durch Ansäuern der Reaktionsmischung und Filtration in an und für sich üblicher Weise isoliert. Durch Zugabe von Alkali und durch Filtration lässt sich die mitentstandene, in Alkali unlösliche Diimid- bzw. Bisbenzimidazol-Verbindung entfernen. Eventuell noch enthaltene, nicht umgesetzte Perylentetracarbonsäure kann über ihr leicht lösliches Tetrakaliumsalz vom meist schwer löslichen Kaliumsalz des erfindungsgemässen Endproduktes abgetrennt werden.

Die erfindungsgemässen neuen Verbindungen der allgemeinen Formel (1) stellen wertvolle Ausgangspunkte zur Herstellung von Farbmitteln (Farbstoffen und Pigmenten), wie von symmetrischen oder asymmetrischen N-substituierten Perylen-3,4,9,10-tetracarbonsäurediimiden (s. bspw. Chemistry Letters 1979, 151–154), dar. Sie können aber auch selbst, gegebenenfalls nach entsprechender Konditionierung, als Farbmittel Verwendung finden, wie beispielsweise in Lakken oder zum Färben von Polymeren, wie Polyolefinen und Polyvinylchlorid.

Die nachfolgenden Beispiele dienen zur näheren Erläuterung der Erfindung. Die Teile sind Gewichtsteile, und die Prozentangaben stellen Gewichtsprozente dar, sofern nicht anders vermerkt. Gewichtsteile verhalten sich zu Volumenteilen wie das Kilogramm zum Liter.

Beispiel 1

39,2 Teile Perylen-3,4,9,10-tetracarbonsäuredianhydrid werden in einer Mischung von 500 Teilen Wasser und 44,5 Teilen Triäthylamin bei 90°C gelöst. Nach Zugabe von 12,3 Teilen 3,5-Dimethylanilin wird mit 85%iger wässriger Phosphorsäure ein pH-Wert zwischen 6,3 und 6,6 eingestellt. Man rührt 6 Stunden bei 90°C weiter und hält hierbei den angegebenen pH-Bereich durch Zutropfen von weiterer Phosphorsäure aufrecht; es werden insgesamt etwa 33 Teile 85%ige Phosphorsäure verbraucht.

Nach Beendigung der Umsetzung werden nochmals 20 Teile 85%ige Phosphorsäure hinzugegeben und das ausgefallene Reaktionsprodukt abgesaugt, zunächst mit 2%iger wässriger Salzsäure und sodann mit Wasser säurefrei gewaschen.

Zur Abtrennung von nicht umgesetzter Perylentetracarbonsäure und mitentstandenem Diimid verfährt man wie folgt: der feuchte Filterkuchen wird in 1500 Teile 5%iger wässriger Kalilauge bei 90 bis 95°C zwei Stunden gerührt, sodann bei 20 bis 25°C abgesaugt und mit 5%iger wässriger Kalilauge bis zum farblosen Filtratablauf gewaschen. Der Filterkuchen wird anschliessend mit heissem Wasser behandelt, wobei das Dikalisalz des Perylentetracarbonsäure-mono-(3,5-dimethyl-phenyl)-imids mit tief rotvioletter Farbe in Lösung geht.

Das unlösliche Diimid wird durch Filtration getrennt, die erfindungsgemässe Verbindung durch Ansäuern des Filtrates ausgefällt und isoliert.

Nach dem Trocknen erhält man die Verbindung der Formel

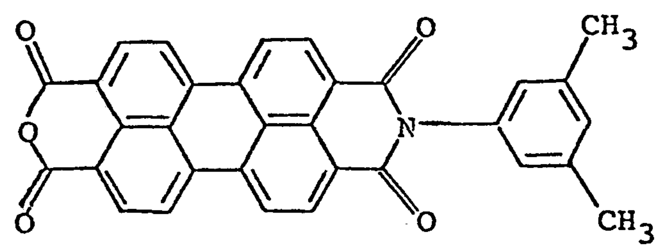

als dunkelrotes Pulver.

Ausbeute: 38,7 Teile (78,2% d.Th.);
Analyse ($C_{32}H_{17}NO_5$):
ber.: C 77,6% H 3,4% N 2,8%
gef.: C 77,3% H 3,6% N 3,0%;
massenspektrographisch: $M^+ = 495$.

Beispiel 1a

Man verfährt in der in Beispiel 1 angegebenen Verfahrensweise, verwendet jedoch anstelle der Phosphorsäure zur pH-Einstellung die entsprechende Menge an konzentrierter wässriger Salzsäure. Man erhält das erfindungsgemässe Produkt in ähnlich guter Qualität in einer Ausbeute von 40% d.Th..

Beispiel 1b

Man verfährt in der in Beispiel 1 angegebenen Verfahrensweise, verwendet jedoch anstelle der Phosphorsäure zur pH-Einstellung die entsprechende Menge an Essigsäure. Man erhält die erfindungsgemässe Verbindung in gleich guter Qualität in einer Ausbeute von 61% d.Th..

Beispiel 2

78,4 Teile Perylen-3,4,9,10-tetracarbonsäuredianhydrid werden in einer Mischung von 800 Teilen Wasser und 89,9 Teilen Triäthylamin bei 90° C gelöst. Man gibt sodann 200 Volumenteile Pyridin und 26,7 Teile 3,5-Dimethyl-anilin hinzu, kühlt auf 20 bis 25° C ab und stellt bei dieser Temperatur mit 85%iger wässriger Phosphorsäure einen pH-Wert von 6,5 ein. Danach erwärmt man den Reaktionsansatz unter Einhaltung eines pH-Bereiches von 6,3 bis 6,6 auf 90° C und führt die Reaktion 6 Stunden bei dieser Temperatur und innerhalb dieses pH-Bereiches durch. Insgesamt

werden etwa 82 Teile 85%ige wässrige Phosphorsäure verbraucht.

Nach Beendigung der Reaktion destilliert man 400 Volumenteile eines Pyridin/Wasser-Gemisches aus dem Ansatz ab, stellt diesen Ansatz sodann oberhalb von 80° C mit konzentrierter wässriger Salzsäure sauer und isoliert das erfindungsgemässe Monoxylidid unter Abtrennung von mitentstandenem Diimid und von der Perylentetracarbonsäure analog der im Beispiel 1 beschriebenen Verfahrensweise.

Man erhält die in Beispiel 1 angegebene erfindungsgemässe Verbindung in gleicher Qualität in einer Ausbeute von 89,9 Teilen (entsprechend 90,8% d.Th).

Beispiel 3

Eine bei 90° C hergestellte Lösung des Tetrammoniumsalzes aus 39,2 Teilen Perylen-3,4,9,10-tetracarbonsäuredianhydrid und 38,3 Teilen Morpholin in 400 Teilen Wasser wird mit einer Lösung von 13,3 Teilen 3,5-Dimethyl-anilin in 200 Volumenteilen Pyridin vereinigt. Zu diesem Reaktionsansatz gibt man bei 90° C innerhalb von 6 Stunden zur Einstellung und Aufrechterhaltung eines pH-Bereiches von 7 bis 6 etwa 40 Teile Essigsäure.

Nach beendeter Umsetzung werden aus dem Reaktionsansatz 400 Volumenteile Flüssigkeit unter gleichzeitiger Zugabe von 200 Teilen Wasser abdestilliert; man säuert mit konzentrierter wässriger Salzsäure an und arbeitet den Ansatz in der im Beispiel 1 angegebenen Verfahrensweise auf.

Man erhält das erfindungsgemässe Monoxylidid in etwa gleich guter Qualität und einer Ausbeute von 81,8% d.Th..

Beispiel 3a

Verfährt man in der im Beispiel 3 angegebenen Verfahrensweise, setzt jedoch anstelle des Tetramorpholiniumsalzes der Perylentetracarbonsäure deren Tetrapiperidinium- oder Tetra-(trimethylammonium)-Salz ein, so erhält man das erfindungsgemässe, im Beispiel 1 formelmässig angegebene Monoxylidid in gleich guter Qualität und etwa gleich hoher Ausbeute.

Beispiel 4

Zu einer bei 80° C hergestellten Lösung von 78,4 Teilen Perylen-3,4,9,10-tetracarbonsäuredianhydrid in 740 Teilen Wasser und 100 Teilen 40%igem wässrigem Dimethylamin lässt man eine Lösung von 20,5 Teilen Anilin in 200 Volumenteilen 4-Picolin zulaufen. Bei einer Temperatur von 20 bis 25° C stellt man dann durch Zugabe von 85%iger wässriger Phosphorsäure einen pH-Wert von 6,5 ein und führt die Umsetzung durch Erhitzen bei 90% C während 3 Stunden unter Aufrechterhaltung eines pH-Bereiches von 6,7 bis 6,3 zu Ende.

Die erfindungsgemässe Monoanilid-monoanhydrid-Verbindung entsprechend der allgemeinen Formel (1a)

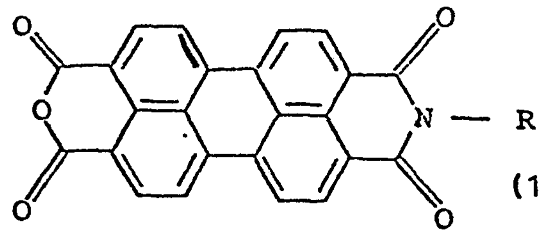

(1a)

in welcher R hier für den Phenylrest steht, wird analog der in Beispiel 1 angegebenen Verfahrensweise aus dem Reaktionsansatz abgetrennt und isoliert.

Ausbeute: 60,3 Teile (64,6% d.Th.);
Analyse ($C_{30}H_{13}NO_5$):
ber.: N 3,0%, gef.: N 3,2%;
massenspektrographisch: $M^+ = 467$.

Beispiel 5

Eine aus 196 Teilen Perylen-3,4,9,10-tetracarbonsäuredianhydrid, 2000 Teilen Wasser und 222 Teilen Triäthylamin bereitete Lösung des Tetra-(triäthylammonium)-Salzes der Perylentetracarbonsäure wird mit 1000 Volumenteilen Pyridin und sodann mit 75,5 Teilen 4-Äthoxy-anilin versetzt und auf 90° C erwärmt. Man stellt mit etwa 40 Teilen Essigsäure den Anfangs-pH-Wert von 8,2 auf 7,0 ein und gibt im Verlaufe von 6 Stunden langsam weitere 160 Teile Essigsäure bei der Reaktionstemperatur von 90° C hinzu, wobei der pH-Wert von 7,0 auf 6,0 absinkt.

Nach Beendigung der Reaktion werden 2000 Volumenteile Flüssigkeit unter gleichzeitiger Ergänzung durch 1000 Teile Wasser aus dem Ansatz abdestilliert und sodann 300 Volumenteile wässrige konzentrierte Salzsäure zugesetzt. Aus der sauren Suspension wird analog der im Beispiel 1 angegebenen Verfahrensweise das erfindungsgemässe, dunkelrote Perylentetracarbonsäure-monoanhydrid-mono-(4-äthoxyanilid) abgetrennt.

Ausbeute: 196 Teile (76,7% d.Th.);
Analyse ($C_{32}H_{17}NO_6$):
ber.: N 2,7%, gef.: N 2,9%;
massenspektrographisch: $M^+ = 511$.

Die Konstitution der erfindungsgemässen Verbindung entspricht der im Beispiel 4 angegebenen allgemeinen Formel (1a), in welcher R für den p-Äthoxyphenyl-Rest steht.

Beispiel 6

Zur Herstellung einer erfindungsgemässen Verbindung verfährt man gemäss der Verfahrensweise des Beispieles 5, setzt jedoch anstelle des 4-Äthoxy-anilins 98,9 Teile 4-Methyl-anilin ein. Man erhält in einer Ausbeute von 71,4% d.Th. das Perylen-3,4,9,10-tetracarbonsäure-monoanhydrid-mono-(4-methyl-anilid) entsprechend der im Beispiel 4 angegebenen allgemeinen Formel (1a) mit R gleich dem 4-Methylphenyl-Rest.

Analyse ($C_{31}H_{15}NO_5$):
ber.: N 2,9%, gef.: N 3,2%;
massenspektrographisch: $M^+ = 481$.

Beispiel 7

Eine gemäss Beispiel 2 hergestellte Triäthylammoniumsalzlösung von Perylen-3,4,9,10-tetracarbonsäure wird nach Zugabe einer Lösung von 27,0 Teilen 4-Methoxy-anilin in 100 Volumenteilen Pyridin bei einer Temperatur von 20 bis 25° C mit 78 Teilen 85%iger wässriger Phosphorsäure versetzt; hierbei fällt der pH-Wert von anfangs 9,8 auf 6,3 ab. Man erwärmt die erhaltene gelbe Suspension auf 90° C und führt die Umsetzung bei dieser Temperatur noch eine Stunde weiter, wobei der pH-Wert weiterhin zunächst auf etwa 5,5 fällt und sodann wieder auf einen pH-Wert von etwa 6,5 ansteigt.

Nach dieser Umsetzung wird das erfindungsgemässe Perylen-3,4,9,10-tetracarbonsäure-monoanhydrid-mono-(4-methoxy-anilid), das der in Beispiel 4 angegebenen allgemeinen Formel (61a) mit R gleich dem 4-Methoxy-phenyl-Rest entspricht, gemäss der im Beispiel 1 abgegebenen Verfahrensweise aus dem Reaktionsansatz abgetrennt und isoliert.

Ausbeute: 52,8 Teile (53,1% d.Th.);
Analyse: ($C_{31}H_{15}NO_6$):
ber.: N 2,8%, gef.: N 2,6%;
massenspektrographisch: $M^+ = 497$.

Beispiel 8

Zur Herstellung einer erfindungsgemässen Verbindung verfährt man in der im Beispiel 2 angegebenen Verfahrensweise, setzt jedoch anstelle von 3,5-Dimethylanilin als Ausgangskomponente 24,0 Teile 2-Aminophenol ein.

Nach Beendigung der Umsetzung wird die erfindungsgemässe Verbindung wie folgt aus dem Ansatz isoliert und von anderen Produkten abgetrennt: Der Reaktionsansatz wird filtriert und der feuchte Filterkuchen in 1000 Volumenteilen 5%iger wässriger Kalilauge heiss gelöst. Diese dunkelrote Lösung wird mit Essigsäure auf einen pH-Wert zwischen 8,5 und 8,8 eingestellt. Nach Zugabe von 100 Teilen Kaliumacetat lässt man abkühlen, saugt das ausgefallene Produkt ab und wäscht es mit 10%iger Kaliumacetatlösung, bis der Filtratablauf farblos ist. Der Rückstand wird in heissem Wasser gelöst, die Lösung filtriert und das erfindungsgemässe Perylen-3,4,9,10-tetracarbonsäure-monoanhydrid-mono-(2-hydroxyphenyl)-imid, das die Konstitution der im Beispiel 4 angegebenen allgemeinen Formel (1a) mit R gleich dem 2-Hydroxyphenyl-Rest besitzt, mit verdünnter Schwefelsäure ausgefällt und isoliert.

Ausbeute: 80,0 Teile (82,8% d. Th.);
Analyse ($C_{30}H_{13}NO_6$):
ber.: N 2,9%, gef.: N 3,0%;
massenspektrographisch: $M^+ = 483$.

Beispiel 9

27,5 Teile Dibrom-perylen-3,4,9,10-tetracarbonsäuredianhydrid werden in 222 Teilen 10%igem wässrigem Triäthylamin bei 80° C gelöst. Zu dieser Lösung gibt man 6,7 Teile 3,5-Dimethylanilin in 50 Volumenteilen Pyridin hinzu, stellt diesen Ansatz mit 85%iger wässriger Phosphorsäure auf einen pH-Wert zwischen 6,4 und 6,6 ein und rührt den Ansatz noch 10 Stunden bei 90° C unter Aufrechterhaltung dieses pH-Bereiches mittels Phosphorsäure.

Nach Beendigung der Umsetzung wird der Ansatz mit konzentrierter wässriger Salzsäure sauer gestellt und die erfindungsgemässe Verbindung gemäss der im Beispiel 1 beschriebenen Verfahrensweise isoliert.

Ausbeute: 10,6 Teile (32,5% d.Th.);

Analyse ($C_{32}H_{15}Br_2NO_5$):
ber.: Br 24,5%, N 2,1%
gef.: Br 21,8%, N 2,2%;
massenspektrographisch: $M^+ = 653$.

Die hier hergestellte erfindungsgemässe Verbindung hat die folgende Konstitution:

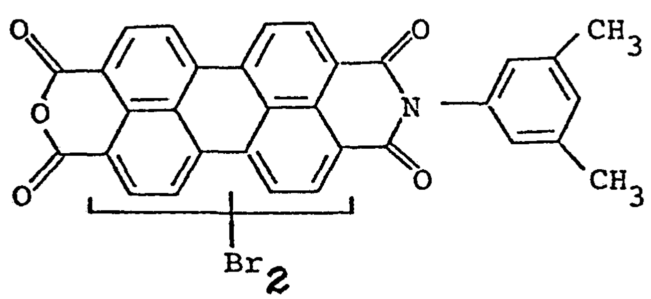

Beispiel 10

39,2 Teile Perylen-3,4,9,10-tetracarbonsäuredianhydrid werden in 500 Teilen Wasser und 44,5 Teilen Triäthylamin heiss gelöst. 400 Volumenteile Pyridin und 32,4 Teile 1,2-Diaminobenzol werden hinzugegeben. Sodann setzt man innerhalb von 2 Stunden bei einer Reaktionstemperatur von 90° C 26 Teile Essigsäure hinzu; hierbei fällt der pH-Wert von anfangs 7,7 auf 5,9 ab. Es wird noch 2 Stunden bei 90° C weitergerührt, wobei der pH-Wert auf etwa 6,4 ansteigt. Nach Abkühlen auf 20 bis 25° C saugt man das dunkelviolette Reaktionsprodukt ab und wäscht es mit Wasser und Methanol salz- und aminfrei.

Zur Isolierung der erfindungsgemässen Verbindung wird der feuchte Filterkuchen in 250 Teilen Wasser angerührt; 40 Teile einer 50%igen wässrigen Kalilauge werden hinzugegeben und das Ganze auf 90° C erwärmt. Hieraus wird durch Zugabe von 60 Teilen Kaliumacetat das Dikaliumsalz des gebildeten Monobenzimidazol-Derivates ausgesalzen. Man saugt es bei 20 bis 25° C ab und wäscht es mit 18%iger wässriger Kaliumacetatlösung, bis es frei von Perylentetracarbonsäure ist. Der Rückstand wird in heissem Wasser gelöst und vom unlöslichen Bis-benzimidazol-Nebenprodukt durch Filtration abgetrennt. Aus dem tiefvioletten Filtrat wird die erfindungsgemässe Verbindung mit Salzsäure sauer ausgefällt und isoliert.

Ausbeute: 31,7 Teile (68,4% d.Th.);
Analyse ($C_{30}H_{12}N_2O_4$):
ber.: N 6,0%, gef.: N 5,8%;
massenspektrographisch: $M^+ = 464$.

Diese erfindungsgemässe Verbindung hat folgende Konstitution:

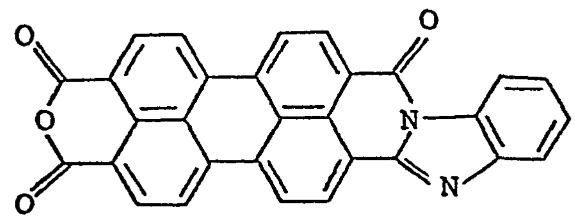

Beispiel 11

Zu einer gemäss Beispiel 2 hergestellten wässrigen Lösung des Perylen-3,4,9,10-tetracarbonsäure-tetra-(triäthyl-ammonium)-Salzes gibt man 200 Volumenteile Pyridin und 34,0 Teile 4-Chlor-1,2-diaminobenzol.
Diesen Reaktionsansatz stellt man bei einer Reaktionstemperatur von 90° C zunächst mit 85%iger wässriger Phosphorsäure auf einen pH-Wert zwischen 6,6 und 6,4 ein und hält diesen pH-Bereich während einer Reaktionszeit von 12 Stunden bei 90° C durch Zugabe von Phosphorsäure aufrecht; hierzu werden insgesamt etwa 72 Teile 85%ige wässrige Phosphorsäure benötigt.

Nach Beendigung der Umsetzung wird der Ansatz wie folgt aufgearbeitet: Man gibt 220 Teile einer 50%igen wässrigen Kalilauge hinzu, destilliert bis zu einer Übergangstemperatur von 98° C ab, kühlt sodann auf 20 bis 25° C herunter, filtriert ab und wäscht den Rückstand mit 18%iger wässriger Kaliumacetatlösung bis zum farblosen Filtratablauf. Der Rückstand wird in heissem Wasser gelöst und das unlösliche Bis-chlorbenzimidazol-Nebenprodukt durch Filtration abgetrennt. Aus dem tiefblauvioletten Filtrat wird das Isomerengemisch der erfindungsgemässen Verbindungen der Formeln

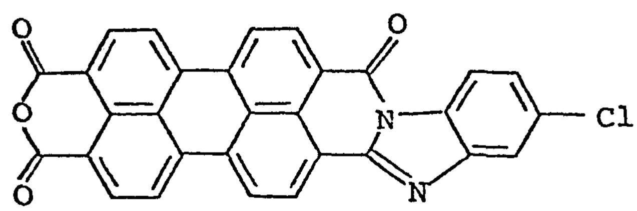

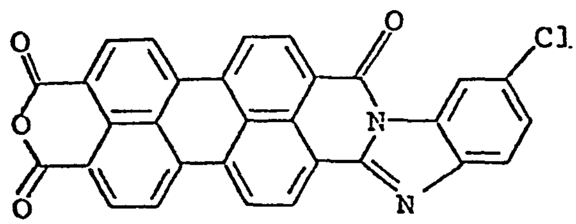

mit Salzsäure ausgefällt, isoliert, mit Wasser gewaschen und getrocknet.

Ausbeute: 44,9 Teile (45% d.Th.);
Analyse ($C_{30}H_{11}ClN_2O_4$):
ber.: Cl 7,1% N 5,6%
gef.: Cl 6,8% N 5,4%;
massenspektrographisch: $M^+ = 498$.

Beispiele 12 bis 28

In den nachfolgenden Tabellenbeispielen sind weitere erfindungsgemässe Verbindungen entsprechend der im Beispiel 4 angegebenen allgemeinen Formel (1a) beschrieben, die in erfindungsgemässer Weise, beispielsweise analog einem der obengenannten Ausführungsbeispiele 1 bis 5 oder 7, durch Umsetzung der erfindungsgemäss eingesetzten Tetrammoniumsalze der Perylen-3,4,9,10-tetracarbonsäure mit den substituierten Anilinverbindungen der Formel $R-NH_2$ hergestellt werden können. Sie sind, wie die in den Beispielen 1 bis 9 beschriebenen erfindungsgemässen Verbindungen, wertvolle Zwischenprodukte zur Herstellung von Farbmitteln und besitzen selbst gute Farbmitteleigenschaften mit guten Echtheiten und anwendungstechnischen Eigenschaften.

| Bsp. | R von Formel (1a) | Analyse<br>ber. (%) | gef. (%) | M+ |
|---|---|---|---|---|
| 12 | 2,5-Dimethyl-phenyl | N 2,8 | N 2,9 | 495 |
| 13 | 3,4-Dimethyl-phenyl | N 2,8 | N 2,5 | 495 |
| 14 | 4-Chlor-phenyl | Cl 7,1<br>N 2,8 | Cl 7,0<br>N 2,9 | 501 |
| 15 | 3-Chlor-phenyl | Cl 7,1<br>N 2,8 | Cl 7,2<br>N 2,9 | 501 |
| 16 | 3-Brom-phenyl | Br 14,6<br>N 2,6 | Br 15,6<br>N 2,5 | 545 |
| 17 | 2,4-Dichlor-phenyl | Cl 13,2<br>N 2,6 | Cl 12,8<br>N 2,7 | 535 |
| 18 | 3-Trifluormethyl-phenyl | F 10,6<br>N 2,6 | F 10,2<br>N 2,6 | 535 |
| 19 | 3,5-Bis-(trifluormethyl)-phenyl | F 18,9<br>N 2,3 | F 18,0<br>N 2,2 | 603 |
| 20 | 2,4,6-Trimethyl-phenyl | N 2,7 | N 2,7 | 509 |
| 21 | 2,5-Dimethoxy-phenyl | N 2,7 | N 2,8 | 527 |
| 22 | 4-(Phenoxy)-phenyl | N 2,5 | N 2,9 | 559 |
| 23 | 4-(Phenylamino)-phenyl | N 5,0 | N 5,0 | 558 |
| 24 | 4-(Phenylazo)-phenyl | N 7,4 | N 7,4 | 571 |
| 25 | Pyrid-3-yl | N 6,0 | N 5,4 | 468 |
| 26 | Pyrid-2-yl | N 6,0 | N 5,8 | 468 |
| 27 | 3-Hydroxy-phenyl | N 2,9 | N 2,6 | 483 |
| 28 | 3-Nitro-phenyl | N 5,5 | N 5,3 | 512 |
| 29 | 4-Brom-phenyl | Br 14,6<br>N 2,6 | N 14,9<br>2,4 | 545 |

Beispiele 30 und 31

In den Tabellenbeispielen 30 und 31 sind weitere erfindungsgemässe Verbindungen beschrieben, die in erfindungsgemässer Weise, beispielsweise analog dem Beispiel 10 oder 11, durch Umsetzung der erfindungsgemäss eingesetzten Tetraammoniumsalze der Perylen-3,4,9,10-tetracarbonsäure mit 4-Methyl-1,2-diaminobenzol für das Isomerengemisch des Beispieles 30 bzw. mit 4,5-Dichlor-1,2-diaminobenzol für die Verbindung des Beispieles 31 hergestellt werden können. Sie sind, wie die in den Beispielen 10 und 11 beschriebenen erfindungsgemässen Verbindungen, wertvolle Zwischenprodukte zur Herstellung von Farbmitteln und besitzen selbst gute Farbmitteleigenschaften mit guten anwendungstechnischen Eigenschaften und Echtheiten.

Beispiel: Verbindung der allgemeinen Formel (1)

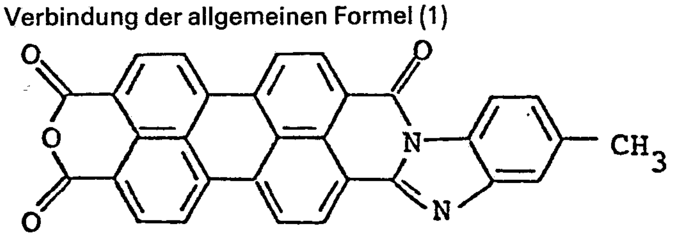

+ Isomeres

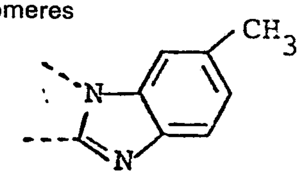

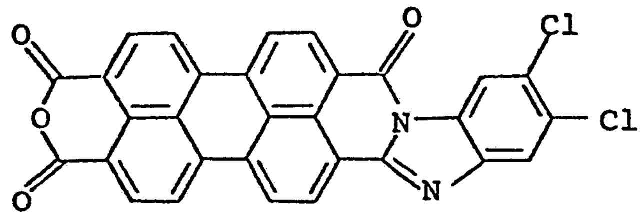

Analysen:

| Bsp. | ber. (%) | gef. (%) | M+ |
|---|---|---|---|
| 30 | N 5,9 | N 5,3 | 478 |
| 31 | Cl 13,3<br>N 5,3 | Cl 12,8<br>N 5,2 | 532 |

**Patentansprüche**

1. Perylen-3,4,9,10-tetracarbonsäure-monoanhydrid-monoimid-Verbindungen der allgemeinen Formel (1)

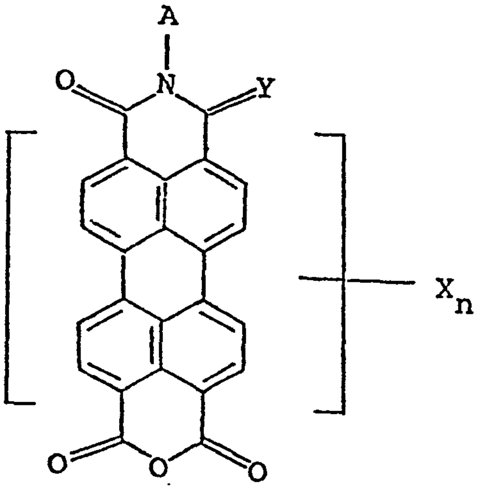

(1)

mit folgender Bedeutung:
X ist ein Chlor- oder Bromatom;
n ist eine Zahl von Null bis 4;
Y steht für ein Sauerstoffatom oder ein Stickstoffatom;
A ist, sofern Y für ein Sauerstoffatom steht, der Phenylrest, der durch Substituenten aus der Gruppe Halogen, Alkyl von 1 bis 4 C-Atomen, Alkoxy von 1 bis 4 C-Atomen, Carbalkoxy mit 1 bis 4 C-Atomen im Alkylrest, Hydroxy, Carboxy, Trifluormethyl, Nitro, Phenoxy, durch Alkyl von 1 bis 4 C-Atomen, Alkoxy von 1 bis 4 C-Atomen und/oder Halogen substituiertes Phenoxy, Phenylamino, im Phenylrest durch Alkyl von 1 bis 4 C-Atomen, Alkoxy von 1 bis 4 C-Atomen und/oder Halogen substituiertes Phenylamino, Phenylazo und durch Alkyl von 1 bis 4 C-Atomen, Alkoxy von 1 bis 4 C-Atomen und/oder Halogen substituiertes Phenylazo substituiert sein kann, oder ist der Pyridylrest, der durch Halogen und/oder Alkyl von 1 bis 4 C-Atomen substituiert sein kann, oder A ist, sofern Y für ein Stickstoffatom steht, der ortho-Phenylenrest, der zusammen mit Y und dem anderen Stickstoffatom den Benzimidazol-Ring bildet, wobei der Benzolkern des Benzimidazolringes durch Substituenten aus der Gruppe Halogen, Alkyl von 1 bis 4 C-Atomen und Alkoxy von 1 bis 4 C-Atomen substituiert sein kann.

2. Verbindungen nach Anspruch 1, dadurch gekennzeichnet, dass Y ein Sauerstoffatom ist und A den Phenylrest bedeutet, der durch Substituenten aus der Gruppe Alkyl von 1 bis 4 C-Atomen, Alkoxy von 1 bis 4 C-Atomen, Chlor und Brom substituiert sein kann.

3. Verbindungen nach Anspruch 1, dadurch gekennzeichnet, dass Y für ein Stickstoffatom steht und A den o-Phenylenrest bedeutet, der durch einen oder zwei Substituenten aus der Gruppe Alkyl von 1 bis 4 C-Atomen, Alkoxy von 1 bis 4 C-Atomen und Chlor substituiert sein kann.

4. Verbindungen nach einem der Ansprüche 1 bis 3, dadurch gekennzeichnet, dass n die Zahl Null bedeutet.

5. Verbindungen nach Anspruch 1, dadurch gekennzeichnet, dass n die Zahl Null bedeutet und A der 4-Brom-phenyl-Rest ist.

6. Verbindungen nach Anspruch 1, dadurch gekennzeichnet, dass Y ein Sauerstoffatom ist, n die Zahl Null bedeutet und A der p-Methoxy- oder p-Äthoxy-phenyl-Rest ist.

7. Verbindungen nach Anspruch 1, dadurch gekennzeichnet, dass Y ein Sauerstoffatom ist, n die Zahl Null bedeutet und A der 3,5-Dimethylphenyl-Rest ist.

8. Verbindungen nach Anspruch 1, dadurch gekennzeichnet, dass Y ein Sauerstoffatom ist, n die Zahl Null bedeutet und A der 4-Chlor-phenyl-Rest ist.

9. Verbindungen nach Anspruch 1, dadurch gekennzeichnet, dass Y ein Sauerstoffatom ist, n die Zahl Null bedeutet und A ein Methylphenyl-Rest ist.

10. Verfahren zur Herstellung der Verbindungen von Anspruch 1, dadurch gekennzeichnet, dass man das Tetrasalz der Perylen-3,4,9,10-tetracarbonsäure der allgemeinen Formel (2)

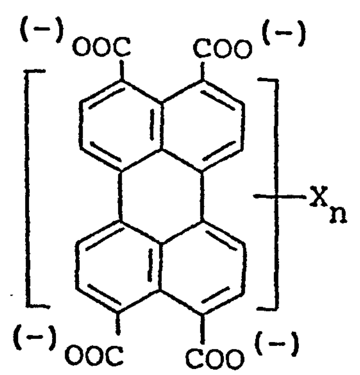

$\cdot\ 4\ M^{(+)}$

(2)

in welcher X und n die in Anspruch 1 genannten Bedeutungen haben und $M^{(+)}$ das Ammoniumion eines sekundären oder tertiären Amins ist, das in wässriger Lösung einen pH-Wert von 8,5 oder höher einzustellen vermag, mit einer aromatischen Aminoverbindung der allgemeinen Formel (3) oder (4)

$B\text{-}NH_2$ (3) $H_2N\text{-}D\text{-}NH_2$ (4)

in welchen B den in Anspruch 1 für A genannten, gegebenenfalls substituierten Phenyl- oder Pyridyl-Rest bedeutet und D den ortho-Phenylenrest darstellt, der durch Substituenten aus der Gruppe Halogen, Alkyl von 1 bis 4 C-Atomen und Alkoxy von 1 bis 4 C-Atomen substituiert sein kann, in wässriger oder wässrig-organischer Lösung miteinander umsetzt und hierbei diese Reaktionslösung zunächst durch Zugabe von Säure auf einen pH-Wert zwischen 7 und 5 einstellt und sodann die Umsetzung bei erhöhter Temperatur innerhalb des pH-Bereiches von 7 bis 5 durchführt.

11. Verfahren nach Anspruch 10, dadurch gekennzeichnet, dass man die Umsetzung bei einer

Temperatur zwischen 70° C und Siedetemperatur des Reaktionsmediums durchführt.

12. Verfahren nach Anspruch 10 oder 11, dadurch gekennzeichnet, dass man die Umsetzung in einem wässrig-organischen Medium durchführt, wobei das organische Medium ein ungesättigtes tertiäres Amin ist.

13. Verfahren nach Anspruch 12, dadurch gekennzeichnet, dass das ungesättigte tertiäre Amin ein Picolin oder Pyridin ist.

14. Verfahren nach Anspruch 12 oder 13, dadurch gekennzeichnet, dass das wässrig-organische Medium des ungesättigte tertiäre Amin in einer Menge von 10 bis 80 Gew.-%, bezogen auf die Wassermenge im Reaktionsmedium, enthält.

15. Verfahren nach einem der Ansprüche 10 bis 14, dadurch gekennzeichnet, dass das Ammoniumion der Ausgangsverbindung der allgemeinen Formel (2) ein Ammoniumion der allgemeinen Formel (5) ist

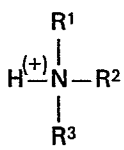

(5)

in welcher $R^1$, $R^2$ und $R^3$ zueinander gleiche oder voneinander verschiedene Bedeutungen haben können und $R^1$ für ein Wasserstoffatom, eine Alkylgruppe von 1 bis 4 C-Atomen oder eine durch eine Hydroxygruppe substituierte Alkylgruppe von 1 bis 4 C-Atomen bedeutet, $R^2$ eine Alkylgruppe von 1 bis 4 C-Atomen bedeutet, die durch eine Hydroxygruppe substituiert sein kann, und $R^3$ eine Alkylgruppe von 1 bis 4 C-Atomen ist, die durch eine Hydroxygruppe substituiert sein kann, oder in welcher $R^2$ und $R^3$ zusammen mit dem Stickstoffatom einen gesättigten, heterocyclischen, gegebenenfalls ein Sauerstoffatom oder Stickstoffatom als weiteres Heteroatom enthaltenden Ring bilden.

16. Verwendung der Verbindungen von Anspruch 1 als Farbmittel.

17. Verwendung der Verbindungen nach Anspruch 1 als Zwischenprodukte zur Herstellung von asymmetrisch N,N'-disubstituierten Perylen-3,4,9,10-tetracarbonsäure-diimiden.

**Claims:**

1. A perylene-3,4,9,10-tetracarboxylic acid mono-anhydride monoimide compound of the formula (1)

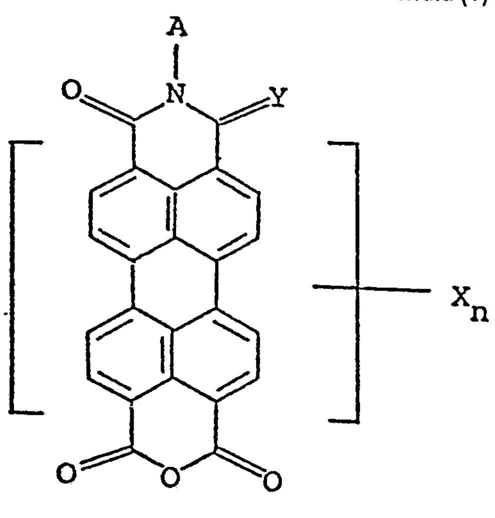

(1)

with the following meanings:
X is a chlorine or bromine atom;
n is a number from zero to 4;
Y represents an oxygen atom or a nitrogen atom;
A, if Y represents an oxygen atom, is the phenyl radical, which can be substituted by substituents from the group comprising halogen, alkyl of 1 to 4 carbon atoms, alkoxy of 1 to 4 carbon atoms, carbalkoxy with 1 to 4 carbon atoms in the alkyl radical, hydroxyl, carboxyl, trifluoromethyl, nitro, phenoxy, phenoxy which is substituted by alkyl of 1 to 4 carbon atoms, alkoxy of 1 to 4 carbon atoms and/or halogen, phenylamino, phenylamino which is substituted in the phenyl radical by alkyl of 1 to 4 carbon atoms, alkoxy of 1 to 4 carbon atoms and/or halogen, phenylazo and phenylazo which is substituted by alkyl of 1 to 4 carbon atoms, alkoxy of 1 to 4 carbon atoms and/or halogen, or A is the pyridyl radical, which can be substituted by halogen, and/or alkyl of 1 to 4 carbon atoms, or

A, if Y represents a nitrogen atom, is the orthophenylene radical, which, together with Y and the other nitrogen atom, forms the benzimidazole ring, it being possible for the benzene nucleus of the benzimidazole ring to be substituted by substituents from the group comprising halogen, alkyl of 1 to 4 carbon atoms and alkoxy of 1 to 4 carbon atoms.

2. Compounds as claimed in claim 1, in which Y is an oxygen atom and A is the phenyl radical, which can be substituted by substituents from the group comprising alkyl of 1 to 4 carbon atoms, alkoxy of 1 to 4 carbon atoms, chlorine and bromine.

3. Compounds as claimed in claim 1, in which Y represents a nitrogen atom and A is the o-phenylene radical, which can be substituted by one or

two substituents from the group comprising alkyl of 1 to 4 carbon atoms, alkoxy of 1 to 4 carbon atoms and chlorine.

4. Compounds as claimed in any one of claims 1 to 3, in which n denotes the number zero.

5. Compounds as claimed in claim 1, in which n denotes the number zero and A is the 4-bromophenyl radical.

6. Compounds as claimed in claim 1, in which Y is an oxygen atom, n denotes the number zero and A is the p-methoxy- or p-ethoxy-phenyl radical.

7. Compounds as claimed in claim 1, in which Y is an oxygen atom, n denotes the number zero and A is the 3,5-dimethyl-phenyl radical.

8. Compounds as claimed in claim 1, in which Y is an oxygen atom, n denotes the number zero and A is the 4-chloro-phenyl radical.

9. Compounds as claimed in claim 1, in which Y is an oxygen atom, n denotes the number zero and A is a methylphenyl radical.

10. A process for the preparation of compounds as claimed in claim 1, which comprises reacting the tetrasalt of perylene-3,4,9,10-tetracarboxylic acid of the general formula (2)

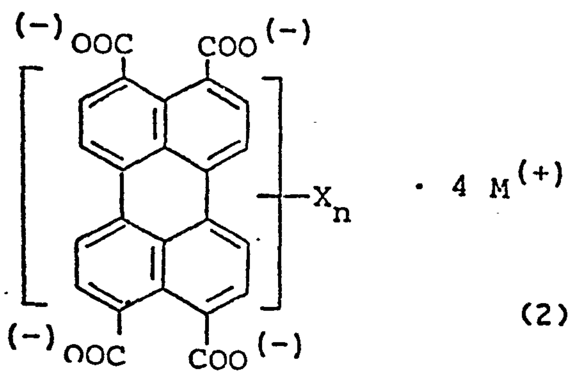

(2)

in which X and n have the meanings given in claim 1 and $M^{(+)}$ is the ammonium ion of a secondary of tertiary amine which is capable of establishing a pH value of 8,5 or higher in aqueous solution, and an aromatic amino compound of the general formula (3) or (4)

$B\text{-}NH_2$ (3) $H_2N\text{-}D\text{-}NH_2$ (4)

in which B denotes the optionally substituted phenyl or pyridyl radical mentioned in claim 1 for A, and D represents the ortho-phenylene radical which can be substituted by substituents from the group comprising halogen, alkyl of 1 to 4 carbon atoms and alkoxy of 1 to 4 carbon atoms, with one another in aqueous or aqueous-organic solution, this reaction solution first being adjusted to a pH value of between 7 and 5 by addition of acid and the reaction then being carried out a elevated temperature within the pH range of 7 to 5.

11. The process as claimed in claim 10, wherein the reaction is carried out at a temperature between 70° C and the boiling point of the reaction medium.

12. The process as claimed in either of claims 10 and 11, wherein the reaction is carried out in an aqueous-organic medium, the organic medium being an unsaturated tertiary amine.

13. The process as claimed in claim 12, wherein the unsaturated tertiary amine is a picoline or pyridine.

14. The process as claimed in either of claims 12 and 13, wherein the aqueous-organic medium contains the unsaturated tertiary amine in an amount of 10 to 80% by weight, based on the amount of water in the reaction medium.

15. The process as claimed in any one of claims 10 to 14, wherein the ammonium ion of the starting compound of the general formula (27) is an ammonium ion of the general formula (5)

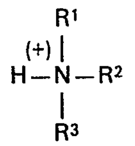

(5)

in which $R^1$, $R^2$ and $R^3$ can have meanings which are identical to one another or different from one another, and $R^1$ denotes a hydrogen atom, an alkyl group of 1 to 4 carbon atoms or an alkyl group of 1 to 4 carbon atoms which is substituted by a hydroxyl group, $R^2$ denotes an alkyl group of 1 to 4 carbon atoms, which can be substituted by hydroxyl group, and $R^3$ is an alkyl group of 1 to 4 carbon atoms, which can be substituted by a hydroxyl group, or in which $R^2$ and $R^3$, together with the nitrogen atom, form a saturated, heterocyclic ring which optionally contains an oxygen atoms or a nitrogen atom as a further hetero-atom.

16. The use of compounds as claimed in claim 1 as colorants.

17. The use of compounds as claimed in claim 1 as intermediates for the preparation of asymmetrically N,N'-disubstituted perylene-3,4,9,10-tetracarboxylic acid diimides.

**Revendications**

1. Composés monoanhydrides-monoimides de l'acide pérylènetétracarboxylique-3,4,9,10 de formule générale (1)

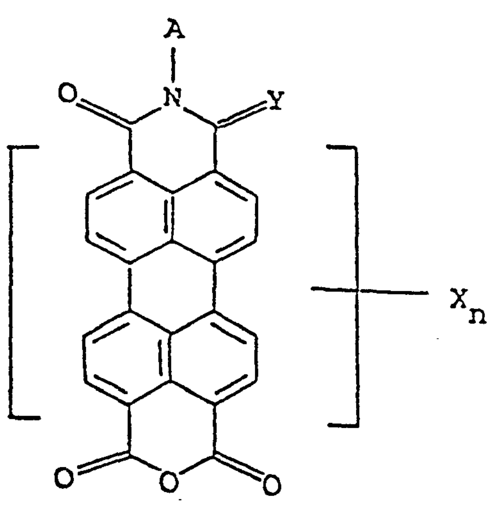

(1)

dans laquelle

X est un atome de chlore ou de brome;

n est un nombre de 0 à 4;

Y représente un atome d'oxygène ou un atome d'azote;

A, si Y est un atome d'oxygène, représente le radical phényle, lequel peut être substitué par des substituants du groupe comprenant les halogènes, les radicaux alkyle ayant de 1 à 4 atomes de carbone, alcoxy ayant de 1 à 4 atomes de carbone, carbalcoxy ayant de 1 à 4 atomes de carbone dans le fragment alkyle, hydroxy, carboxy, trifluorométhyle, nitro, phénoxy, phénoxy substitué par un radical alkyle ayant de 1 à 4 atomes de carbone ou un radical alcoxy ayant de 1 à 4 atomes de carbone et/ou un halogène, phénylamino, phénylamino substitué dans le fragment phényle par un radical alkyle ayant de 1 à 4 atomes de carbone ou par un fragment alcoxy ayant de 1 à 4 atomes de carbone et/ou par un halogène, phénylazo ou phénylazo substitué par un radical alkyle ayant de 1 à 4 atomes de carbone ou par un radical alcoxy ayant de 1 à 4 atomes de carbone et/ou par un halogène, ou bien représente le radical pyridyle, lequel peut être substitué par un halogène et/ou par un radical alkyle ayant de 1 à 4 atomes de carbone, ou bien A, si Y est un atome d'azote, représente le radical ortho-phénylène qui, ensemble avec Y et l'autre atome d'azote, forme le noyau benzimidazole, le noyau benzénique du cycle benzimidazole pouvant être substitué par des substituants du groupe comprenant les halogènes, les radicaux alkyle ayant de 1 à 4 atomes de carbone et alcoxy ayant de 1 à 4 atomes de carbone.

2. Composés selon la revendication 1, caractérisés en ce que Y est un atome d'oxygène, et A représente le radical phényle, qui peut être substitué par des substituants du groupe comprenant les radicaux alkyle ayant de 1 à 4 atomes de carbone, alcoxy ayant de 1 à 4 atomes de carbone, chlore et brome.

3. Composés selon la revendication 1, caractérisés en ce que Y est un atome d'azote et A représente le radical o-phénylène, qui peut être substitué par un ou deux substituants du groupe comprenant les radicaux alkyle ayant de 1 à 4 atomes de carbone, alcoxy ayant de 1 à 4 atomes de carbone, et chlore.

4. Composés selon l'une des revendications 1 à 3, caractérisés en ce que n représente le nombre zéro.

5. Composés selon la revendication 1, caractérisés en ce que n représente le nombre zéro et A est le radical bromo-4 phényle.

6. Composés selon la revendication 1, caractérisés en ce que Y est un atome d'oxygène, n représente le nombre zéro et A est le radical p-méthoxy- ou p-éthoxy-phényle.

7. Composés selon la revendication 1, caractérisés en ce que Y est un atome d'oxygène, n représente le nombre zéro et A est le radical diméthyl-3,5 phényle.

8. Composés selon la revendication 1, caractérisés en ce que Y est un atome d'oxygène, n représente le nombre zéro et A est le radical chloro-4 phényle.

9. Composés selon la revendication 1, caractérisés en ce que Y est un atome d'oxygène, n représente le nombre zéro et A est un radical méthylphényle.

10. Procédé pour la préparation de composés selon la revendication 1, caractérisé en ce qu'on fait réagir l'un sur l'autre, en solution aqueuse ou aqueuse-organique, le tétrasel de l'acide pérylènetétracarboxylique-3,4,9,10 de formule générale (2)

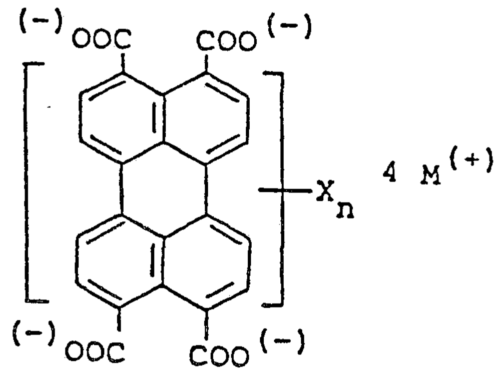

(2)

dans laquelle X et n ont les significations données dans la revendication 1 et $M^{(+)}$ représente l'ion ammonium d'une amine secondaire ou tertiaire, dont on peut ajuster le pH en solution aqueuse à 8,5 ou plus, sur un composés aminé aromatique de formule générale (3) ou (4)

$$B\text{-}NH_2 \quad (3) \qquad H_2N\text{-}D\text{-}NH_2 \quad (4)$$

où B représente le radical phényle ou pyridyle correspondant à A dans la revendication 1, éventuellement substitué, et D représente le radical ortho-phénylène, qui peut être substitué par des substituants du groupe comprenant les halogènes, les radicaux alkyle ayant de 1 à 4 atomes de carbone et alcoxy ayant de 1 à 4 atomes de carbone, puis qu'on ajuste le pH de cette solution réactionelle, d'abord par addition d'acide, à une valeur comprise entre 7 et 5, puis qu'on met en œuvre à haute température dans l'intervalle de pH de 7 à 5.

11. Procédé selon la revendication 10, caractérisés en ce qu'on met en œuvre la réaction à une température comprise entre 70° C et le point d'ébullition du milieu réactionnel.

12. Procédé selon la revendication 10 ou 11, caractérisé en ce qu'on met en œuvre la réaction dans un milieu aqueux-organique, le milieu organique étant une amine tertiaire insaturée.

13. Procédé selon la revendication 12, caractérisé en ce que l'amine tertiaire insaturée est une picoline ou une pyridine.

14. Procédé selon la revendication 12 ou 13, caractérisé en ce que le milieu aqueux-organique contient l'amine tertiaire insaturée en une quantité de 10 à 80% en poids par rapport à la quantité d'eau dans le milieu réactionnel.

15. Procodé selon l'une des revendications 10 à 14, caractérisé en ce que l'ion ammonium du composé de départ de formule générale (2) est un ion ammonium de formule générale (5)

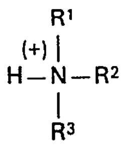

(5)

dans laquelle $R^1$, $R^2$ et $R^3$ peuvent avoir des significations identiques les unes aux autres ou différentes les unes des autres, et $R^1$ est un atome d'hydrogène, un groupe alkyle ayant de 1 à 4 atomes de carbone ou un groupe alkyle ayant de 1 à 4 atomes de carbone et substitué par un groupe hydroxy, $R^2$ est un groupe alkyle ayant de 1 à 4 atomes de carbone, pouvant être substitué par un groupe hydroxy, et $R^3$ est un groupe alkyle ayant de 1 à 4 atomes de carbone et pouvant être substitué par un groupe hydroxy, ou bien dans laquelle $R^2$ et $R^3$ forment ensemble avec l'atome d'azote un noyau hétérocyclique saturé, contenant éventuellement comme hétéroatome supplémentaire un atome d'oxygène ou un atome d'azote.

16. Utilisation des composés selon la revendication 1 en tant que colorants.

17. Utilisation des composés selon la revendication 1 en tant que produits intermédiaires pour la préparation de diimides N,N'-disubstitués et asymétriques de l'acide pérylènetétracarboxylique-3,4,9,10.